# EUROPEAN PATENT APPLICATION

(11) **EP 1 426 043 A1**
(43) Date of publication of application: **09.06.2004**
(21) Application number: 03078404.5
(22) Date of filing: 30.10.2003
(51) Int. Cl.: A61K 9/10, A61K 9/14

(54) **Effervescent pharmaceutical compositions for non conventional suspensions**

(30) Priority: 07.11.2002 IT mi20022358
(71) Applicant: B.S.D., 20060 Bussero (Milan) (IT)
(72) Inventor: Omini, Claudio, 20060 Bussero (IT); Zuccari, Giuseppe, 26866 Sant'Angelo Lodigiano (IT)

(57) **Abstract**

The present invention discloses pharmaceutical compositions comprising hydrocolloids and effervescent agents to obtain extemporaneous homogeneous suspensions of one or more active compounds, particularly useful for nutritional supply of patients suffering of the hepatic diseases or for weight loss in patients in the need.

## Description

### Background of the invention

Suspensions are heterogeneous dispersions where the disperse phase is solid particles, and the dispersing phase is a liquid.

In pharmacy, suspensions are useful when the active compound is insoluble or poor soluble in the desired vehicle, generally water, or when it is unstable in solution, by avoiding or at least reducing the hydrolytic phenomenon (1).

Moreover, suspension may result in slow release of active compounds as compared to solutions, due to the possible modifications in the drug absorption.

A number of chemic-physical factors may influence the shelf-life of the suspensions such as the homogeneity during time without caking, an eventuality which may alter the dosing of the formulate (2).

In the conventional suspensions the disperse particles tend to sediment on the bottom following the well known rule of Stockes (3). Therefore, in order to maintain the homogeneity of the dosing, the formulate should follow the following requirement:
a) The sedimentation velocity should be slow and constant during time.
b) The possible sediment should be eventually easily and homogenously dispersed by shaking.
c) After shaking the formulation should be homogonously and easily flowing from the bottle.

The difficulties or the impossibility to maintain for long time the desired technical characteristics of the pharmaceutical suspensions such as homogeneity and stability may be overcome by the use of the extemporaneous suspensions. This classical pharmaceutical technique allows obtaining the suspension by the addition of a fixed dose of liquid to a pre-dosed amount of powder or granulates, and it has to be drunk until the rheological characteristics are acceptable.

In this regard, the multidose suspensions (i.e. antibiotics) are easily obtained by the conventional suspension technique, and every administration are restored by strong shaking before use.

Monodose suspensions might be obtained by the same technique, but this procedure is unused since in practice it should require several bottles to be strongly shaken with enough volume of water. These bottles should be also packaged in secondary packages which are obviously expensive and cumbersome, so that they are not preferred by distributors because this requires adjunctive costs.

Therefore, the modem pharmacy prefers monodose in sachets or blisters to be dissolved in a glass of water with strong stirring of the formulate. This easy procedure requires that the patient shakes the formulate for at least 1 min in order to obtain a homogeneous suspension; but, as reported in WO 9200731, this simple procedure is generally not followed by the patients and the shaking time of the suspension did not exceed 20-30 sec.

There are patents claiming achievement of homogeneous suspensions within 30 sec. (WO 9200731 and WO 9817250).

### Aim of the invention

The present invention provides pharmaceutical formulations particularly useful for extemporaneous, but not solely, non conventional suspensions; moreover additional aim of the present invention is to obtain a new pharmaceutical form for oral administration of water insoluble or poor soluble compounds, and able to overcome the previous reported technical limitations.

Within the aim of the present invention there are pharmaceutical compositions in powder or granulates for oral solutions/suspensions which, after mixing an adequate amount of water or other drinkable liquids, result in a preferable extemporaneous suspension, where the structural phase of the vehicle, showed by the increase of viscosity from <500 mPa.s to >3000 mPa.s, follows the dispersion phase of the components.

The present invention further contemplates pharmaceutical compositions in powder or granulates for oral solutions/suspensions which, after mixing an adequate amount of water or other drinkable liquids, result in a more preferable extemporaneous suspension, where the structural phase of the vehicle follows the dispersion phase of the components by the aid of an effervescent reaction.

Furthermore, within the aim of the present invention there are pharmaceutical compositions in powder or granulates for oral solutions/suspensions which, after mixing an adequate amount of water or other drinkable liquids, result in an extemporaneous homogeneous suspension of a large number of active compounds with highly different degrees of solubility.

The present invention further contemplates pharmaceutical compositions in powder or granulates for oral solutions/suspensions which, after mixing an adequate amount of water or other drinkable liquids, result in an extemporaneous homogeneous suspension within 30 sec. and more preferable between 15 and 20 sec.

The previous and other aims are reached by pharmaceutical compositions in powder or granulates for oral solutions/suspensions including one or more insoluble or poor-soluble active compounds, or a mixture of different active compounds with a highly different degree of solubility or water-dispersion; one or more soluble or water-dispersible hydrocolloids and a couple of effervescent agents able to produce self-structuring suspensions.

The term self-structuring means that the dispersion phase of the excipients and active compounds, and the subsequent achievement of a structured suspension (thickening and/or gelling) will primary be, according to one embodiment of the present invention, a consequence of the reaction of hydrocolloids and effervescent agents.

### Summary of the invention

One embodiment of the present invention concerns the use of simple effervescent reactions which, through a mechanical activity of the generating gas (i.e. CO₂), will allow the complete dispersion of active compounds and excipients.

In another embodiment, adequate water-soluble or dispersible excipients may be incorporated in water or other drinkable liquids resulting in a stabilization of the suspension generated by the abovementioned effervescent reactions.

To obtain the desired self-structuring formulations, one embodiment of the present invention comprises water-soluble or -dispersible hydrocolloids preferably at room temperature, and which do not require heating over 50-60 °C for solution; this requirement is necessary in case of the extemporaneous suspensions and using active compounds sensible to heat.

The term hydrocolloids in the present invention indicate all the hydrophilic biopolymers including poly-electrolytes from plant, animal and chemical or biotechnological origin. The hydrocolloid - water interaction may induce water retention and adhesion, and providing mechanical properties, structured the environment, and stabilising the suspensions.

Hydrocolloids particularly preferred in the present invention are: gelatines, carrageenans celluloses including carboxymethylcellulose, xanthan gum, pectins, alginates, guar gum, starch such as locust bean starch, arabinoxylan, chitosan, and other natural fibres, β-glucans, poloxamers etc.

One embodiment of the present invention is the use of mixtures of different hydrocolloids since it is well known in the art that these mixtures may perform better than the single components.

In this regard particularly preferred in the present invention are the mixtures of locust bean starch and carrageenans and particularly iota-carrageenans in ratio by weight between 1 to 1 and 10 to 1, and particularly preferred are the ratios between 0.5 parts by weight of locust bean starch and 1 part by weight of carrageenans and 7 parts by weight of locust bean starch and 1 part by weight of carrageenans.

The above mentioned hydrocolloids alone or in mixture are added to effervescent agents in water and, surprisingly, the effervescent reaction is able to ensure complete dispersion of both the active compounds and the hydrocolloids, resulting in homogenous suspension.

The possible effervescent agents are well known in the art and include Na and K carbonates and bicarbonates with acid counterparts such as tartaric acid, citric acid, maleic acid, ascorbic acid, fumaric acid and if it is desired also covered, filmed or in any way protected by environmental humidity.

The abovementioned results are particularly surprising, even if it is well known in the art and already claimed by WO 9200731 that the thickening or suspending agents, including hydrocolloids, when mixed to carbonates or bicarbonates in presence of acids and water may result in extemporaneous suspensions.

However, in the abovementioned patent it is clearly claimed that the carbonates or bicarbonates and the acid counterparts must be added to see that the effervescent reaction may not occur.

Furthermore, these authors clearly stated that the effervescent reaction should be avoided in any case, since it will produce the opposite effect than desired.

In the present invention surprising it has been found that, on the contrary to what claimed by the abovementioned patent WO 9200731, the effervescent reaction allows a rapid and homogeneous formation of extemporaneous suspension also in the presence of hydrocolloids.

One embodiment of the present invention comprises the ratio between effervescent agents (carbonates and bicarbonates + acids) and hydrocolloids ranging from 1:0.1 to 1:1 and particularly preferred from 1:0.5 and 1:1, and where the sum of effervescent agents (carbonates and bicarbonates + acids) is always higher or equal to that of hydrocolloids or other suspending or thickening agents.

The above ratio is exactly the opposite to what claimed by the abovementioned patent (WO 9200731) which claims a ratio between effervescent agents (carbonates and bicarbonates + acids) and hydrocolloids or other suspending or thickening agents ranging from 1:1.5 to 1:5 where the amount of the suspending or thickening agents largely exceeding the amount of effervescent agents.

A possible explanation between our results and those found by WO 9200731 might be related to the need in this last patent of effervescent agents in an amount just enough to induce a rapid hydration of the thickener, but not to obtain effervescent reaction.

On the other hand, in the present invention our results clearly indicate the need of the effervescent reaction to obtain a rapid formation of an extemporaneous and homogenous suspension.

Moreover, it is interesting to underline that the effervescent reaction is in some way facilitated by the presence of hydrocolloids.

In this regard mixing NaHCO₃ 250 mg + Na₂CO₃ 250 mg and 396 mg of citric acid in 50-60 ml of water, the effervescent reaction is very poor, whereas adding at the same amount of effervescent agents 700 mg of locust bean starch (Viscogum) + 100 mg of carrageenan (Satiagel) the effervescent reaction is very active and at homogenous suspension is achieved.

The present invention is more advantageous than the abovementioned patent WO 9200731 since it allows obtaining a pharmaceutical acceptable suspension by simply mixing excipients and active compounds both as powders.

On the contrary, in the WO 9200731 it is claimed the need of a direct mixing of acids and carbonates/bicarbonates and the thickeners and suspending agents in the liquid where the active compounds are dissolved. Moreover, when the effervescent agents are insoluble in the solvent of suspending agents, they should be micronised in order to obtain a similar granulometry among the various components.

All these technical disadvantages are overcome by the present invention, and indeed the presence of real technical problems in the above described process is also confirmed by the same authors who, in a subsequent and more recent patent (WO 9817250) overcoming the previous one, claim a new method for producing the granulate, without using inflammable or dangerous for the environment solvents, and using small amount of excipients.

The use of granulates is not required by the present invention, resulting in a clear-cut technical and industrial advantage over the above mentioned patents; however, if required, the use of granulates obtained following standard procedures is also within the aim of the present invention.

### EXAMPLES

The present invention is further described by the following examples however they must be considered only illustrative of this invention, without to be considered possible limitations or restrictions of the object and the scope of said invention.

### Example 1

Locust bean starch (Viscogum® Degussa) 0.4 g; iota-carrageenan (Satiagel 550® Degussa) 0.6 g; citric acid 0.5; Na₂CO₃ 0.5 g; Cassia nomame 0.2 g; sorbitol 3 g; orange flavour 0.2 g; Na cyclamate 0.02 g.
Dissolution time in 50 ml H₂O: 20 sec.

### Example 2

Locust bean starch (Viscogum® Degussa) 0.5 g; iota-carrageenan (Satiagel 550® Degussa) 0.5 g; citric acid 0.6; NaHCO₃ 0.5 g; Monascus 0.5 g; mannitol 2 g; acesulfame K 0.1 g; peppermint flavour 0.2 g; K sorbate 0.3 g.
Dissolution time in 50 ml H₂O: 18 sec.

These two examples referred to formulations useful for anti-obesity and slim compounds or to reduce cholesterol and triglycerides.
These formulations are particularly preferred since allow to obtain homogenous suspensions of poorly soluble or insoluble active compounds in less than 20 sec.

### Example 3

Locust bean starch (Viscogum® Degussa) 0.7 g; iota-carrageenan (Satiagel 550® Degussa) 0.1 g; film-coated citric acid 0.4; NaHCO₃ 0.25 g; Na₂CO₃ 0.25 g; Aerosil 0.06 g; mannitol 0.934 g; Lysine chloride 0.187 g; Threonine 0.05 g; Leucine 0.1 g; Isoleucine 0.06 g; Valine 0.06 g; Arginine 0.03 g; Cysteine 0.05 g; Aspartic acid 0.08 g; Omitine 0.03 g; Glutamine 0.03 g; Thiamine 0.0022 g; Riboflavine 0.0022 g; Nicotinic acid 0.011 g; Pantothenic acid 0.0059 g; Pyridoxine 0.0022 g; Cyanocobalamin 0.00165 g; Biotin 110 mcg; Vitamin C 0.099 g; Vitamin E 0.033 g; Vitamin K 77 mcg; Vitamin D 5.5 mcg; Vitamin A 0.00253 g; Choline bitartrate 0.486 g; Folic acid 110 mcg; Cr picolinate 403 mcg; Mg oxide heavy 0.083 g; Mn gluconate 0.0081 g; Mo aminochelate 0.05 g; KH₂PO₄ 0.174 g; Se proteinate 0.025 g; Zn aminochelate 0.020 g; Ca₃O₈P₂ 0.1806 g; wood fruits flavour 0.4 g; acesulfame K 0.0175; Na cyclamate 0.02 g; E 127 0.004 g.

The formulation in the example 3 is useful for administering to patients with hepatic diseases in the needs: vitamins, minerals and amino acids, and this formulation is particularly preferred since it is the sole complete formulation for integration of the nutritional need of these patients.

It well known in the art the use of vitamins, salts and amino acids for the nutritional supply of patients suffering of hepatic diseases; however, the presence of vitamins, salts and amino acids in doses right for these patients in a single pharmaceutical composition has not previously disclosed because of several technical problems, overcome by the present invention.

Indeed the clear advantage of the present invention allows the administration in a sole formulation of all the compounds needed and adequate for the nutritional supply of the patients suffering of hepatic diseases.

Moreover, the present invention allows obtaining an extemporaneous and homogeneous suspension of a great number of compounds (more than 30) with a highly different degree of solubility and dispersion.

Further advantage of the present invention is the possibility to easily mask the bad or the contrasting taste of the different compounds present in the formulation.

The above mentioned examples if added to a small amount of tap water (about 50 ml) result in a homogeneous suspension within 15-20 sec; if a greater amount of water or other drinkable liquids is desired, a homogeneous solution/suspension is always obtained.

Further embodiment of the present invention is the use of a sole thickening and/or gelling agent instead of a mixture of them, particularly in formulations containing chitosan oligosaccharide with vitamin C and salts thereof, to be used in the therapy of obesity or in the weight loss as in the following example.

### Example 4

Chitosan C 0.33 g; Mg pyruvate 0.5 g; Locust bean starch (Viscogum® Degussa) 0.6 g; citric acid 0.7 g; NaHCO₃ 0.4 g; mannitol 1 g; acesulfame K 0.0175; orange bitter flavour 0.2 g.

It should be underline that in the above reported example the use of carrageenans results in the precipitation of the formulate after the water addition, and therefore in this case it is particularly preferred the use of a sole hydrocolloid such as locust bean starch.

It is self-evident that sweet agents, fragrances, preservatives and other excipients used in the above reported examples may be easily substituted or integrated with other compounds with similar activity.

The formulations may be packaged as monodose dispenser such as sachets or in multidose dispenser.

### REFERENCES

1) Pharmazie 55: 829; 2000
2) Colloids Surf. Bioterfaces 20: 73; 2001
3) Remington's Pharmaceutical Sciences handbook, Mack Publishing Company USA

## Claims

1. Pharmaceutical compositions comprising one or more active compounds insoluble or poorly soluble in water, one or more hydrocolloid soluble or easily dispersible in water, at least a couple of effervescent agents able to prepare self-structuring suspensions.

2. Pharmaceutical compositions according to claim 1 wherein the active compounds have a highly and non-homogeneous degree of solubility or dispersion in water.

3. Pharmaceutical compositions according to claim 1 wherein the hydrocolloids are: gelatines, carrageenans, celluloses including carboxyimethylcellulose, xanthan gum, pectins, alginates, guar gum, starch such as locust bean starch, arabinoxylan, chitosan, and other natural fibres, β-glucans, poloxamers and mixtures thereof.

4. Pharmaceutical compositions according to claim 1 wherein the effervescent agents is a couple of at least one compound selected in each of the following groups:
a) NaHCO₃, Na₂CO₃, KHCO₃, K₂CO₃.
b) Citric acid, tartaric acid, maleic acid, ascorbic acid, fumaric acid and, or industrially preferred derivatives thereof.

5. Pharmaceutical compositions according to claim 1 wherein the ratio in weight between hydrocolloids: effervescent agents are from 1:0.1 to 1:1.

6. Pharmaceutical compositions according to claim 5 wherein the ratio between hydrocolloids: effervescent agents are from 1:0.5 to 1:1.

7. Pharmaceutical compositions according to claim 3 wherein the carrageenans are: iota-carrageenans.

8. Pharmaceutical compositions according to claim 3 wherein the stark is: locust bean starch.

9. Pharmaceutical compositions according to claim 1 wherein the hydrocolloids are: a mixture of two hydrocolloids.

10. Pharmaceutical compositions according to claim 9 wherein the two hydrocolloids are any mixture of locust bean starch and iota-carrageenans in a ratio in weight from 1:1 to 10:1.

11. Pharmaceutical compositions according to claim 10 wherein ratio in weight of locust bean starch and iota-carrageenans are from 0.5:1 to 7:1.

12. Pharmaceutical compositions according to claim 1 wherein in the presence of chitosan, chitosan oligosaccharide and their salts or conjugation with vitamin C or other acceptable chemical counterparts, the hydrocolloid is locust bean starch.

13. Pharmaceutical compositions according to any of the previous claims useful for extemporaneous self-structuring suspensions in presence of water or other drinkable liquids.

14. Pharmaceutical compositions according to claim 13 wherein the hydrocolloid is water-soluble or -dispersible at less than 50 C°.

15. Pharmaceutical compositions according to claim 14 wherein the hydrocolloids are water-soluble or -dispersible at room temperature.

16. Pharmaceutical compositions according to claim 13 wherein the extemporaneous suspension is achieved within 30 sec., and particularly preferred within 15 and 20 sec.

17. Pharmaceutical compositions according to any of the previous claims performed by the sole mixing of the powders.

18. Pharmaceutical compositions according to any of the previous claims comprising granulates or powders packaged in sachets or in multi-dose dispensers.

19. Pharmaceutical compositions according to any of the previous claims comprising as active compounds: Lysine; Threonine; Leucine; Isoleucine; Valine; Arginine; Cysteine; Aspartic acid; Omitine; Glutamine; Thiamine; Riboflavine; Nicotinic acid; Pantothenic acid; Pyridoxine; Cyanocobalamin; Biotin; Vitamin C; Vitamin E; Vitamin K; Vitamin D; Vitamin A; Choline bitartrate; Folic acid; Cr picolinate; Mg oxide heavy; Mn gluconate; Mo aminochelate; KH₂PO₄; Se proteinate; Zn aminochelate; Ca₃O₈P₂.

20. Pharmaceutical compositions according to any of the previous claims useful for nutritional supply of patients suffering of the hepatic diseases in the need.

21. Pharmaceutical compositions according to any claims from 1 to 19 comprising as active compound: Cassia nomame.

22. Pharmaceutical compositions according to any claims from 1 to 19 comprising as active compound: Monascus

23. Pharmaceutical compositions according to any of the previous claims useful for nutritional supply for anti-obesity treatment in patients in the need.

24. Pharmaceutical compositions according to any claims from 1 to 22 useful for nutritional supply for anti-hypercholestolemia and anti-hyperlipidemia treatment in patients in the need.

25. Pharmaceutical compositions according to claim 12 comprising as active compounds: chitosan and Mg piruvate.

26. Pharmaceutical compositions according to any of the previous claims useful for nutritional supply for weight loss treatment in patients in the need.
